# EUROPÄISCHE PATENTSCHRIFT

(11) **EP 1 871 740 B1**
(45) Veröffentlichungstag und Bekanntmachung des Hinweises auf die Patenterteilung: **16.05.2012**
(21) Anmeldenummer: 06708606.6
(22) Anmeldetag: 02.03.2006
(51) Int. Cl.: C07C 319/08, C07C 321/04

(54) **VERFAHREN ZUR KONTINUIERLICHEN HERSTELLUNG VON METHYLMERCAPTAN**
METHOD FOR THE CONTINUOUS PRODUCTION OF METHYL MERCAPTAN
PROCEDE DE PRODUCTION CONTINUE DE METHYLMERCAPTANE

(30) Priorität: 09.04.2005 DE 102005016369
(43) Veröffentlichungstag der Anmeldung: 02.01.2008
(73) Patentinhaber: Evonik Degussa GmbH, 45128 Essen (DE)
(72) Erfinder: BARTH, Jan-Olaf, 60598 Frankfurt am Main (DE); REDLINGSHÖFER, Hubert, 91481 Münchsteinach (DE); WECKBECKER, Christoph, 63584 Gründau-Lieblos (DE); HUTHMACHER, Klaus, 63571 Gelnhausen (DE)
(86) Internationale Anmeldenummer: PCT/EP2006/060403
(87) Internationale Veröffentlichungsnummer: WO 2006/108737

(56) Entgegenhaltungen:
- EP-A- 0 832 687
- EP-A- 0 850 922

## Beschreibung

Die Erfindung betrifft ein Verfahren zur kontinuierlichen Herstellung von Methylmercaptan durch Umsetzen eines Eduktgasgemisches aus Methanol und Schwefelwasserstoff in der Gasphase bei einer Reaktionstemperatur zwischen 200 und 600°C und einem Betriebsdruck von 1,5 bis 40 bar an einem Katalysator in einem Mehrbettreaktor.

Methylmercaptan EP0850922 ist ein industriell wichtiges Zwischenprodukt für die Synthese von Methionin sowie für die Herstellung von Dimethylsulfoxid und Dimethylsulfon. Methylmercaptan wird überwiegend aus Methanol und Schwefelwasserstoff durch Reaktion an einem Katalysator bestehend aus einem Aluminiumoxidträger und Übergangsmetalloxiden und basischen Promotoren hergestellt. Die Synthese des Mercaptans erfolgt gewöhnlich in der Gasphase bei Temperaturen zwischen 300 und 500°C und bei Drücken zwischen 1 und 25 bar. Die Umsetzung von Schwefelwasserstoff und Methanol zu Methylmercaptan ist ein exothermer Prozess. Die DE-C 196 54 515 beschreibt z.B. ein Verfahren zur Darstellung von Methylmercaptan in einem Rohrbündelreaktor, bei dem die freiwerdende Reaktionswärme über eine Salzschmelze abgeführt und dann indirekt über Wärmetauscher zur Verdampfung von Methanol ausgenutzt wird.

Das Produktgasgemisch enthält neben dem gebildeten Methylmercaptan und Wasser die nicht umgesetzten Ausgangsstoffe Methanol und Schwefelwasserstoff und als Nebenprodukte Dimethylsulfid und Dimethylether, sowie in geringen Mengen auch Polysulfide (Dimethyldisulfid). Im Sinne der Reaktion inerte Gase wie beispielsweise Kohlenmonoxid, Kohlendioxid, Stickstoff und Wasserstoff sind auch im Produktgas enthalten.

Aus dem Produktgasgemisch wird das gebildete Methylmercaptan, wie in DE 17 68 826 erläutert, in mehreren Destillations- und Waschkolonnen bei Temperaturen zwischen 10 und 140°C abgetrennt.

Die Reaktion gemäss GB 14 17 532 kann in einem Festbettreaktor mit mehreren Katalysatorbetten oder in mehreren aufeinander folgenden Reaktoren durchgeführt werden. Methylmercaptan wird dabei durch die Reaktion eines Gemisches von Methanol mit Schwefelwasserstoff in einem molaren Verhältnis von 1,10 : 1 und 2,5 : 1 hergestellt, wobei beide Reaktionspartner dem Reaktor getrennt zugeführt werden. Gemäß der FR 24 77 538 wird zur Herstellung von Methylmercaptan frisches Schwefelwasserstoffgas in einem Verdichter auf 11 bar verdichtet. Danach wird aus dem Prozess zurückgeführtes Kreisgas, welches Schwefelwasserstoff, Dimethylsulfid, Methanol und geringe Mengen Methylmercaptan enthält, mit dem verdichteten Schwefelwasserstoff zur Bildung des Eduktgasgemisches gemischt und dieses auf 510°C aufgeheizt. Vor Eintritt in den ersten von bis zu 10 hintereinandergeschalteten Reaktoren, wird dem Eduktgasgemisch der Waschmittelkreisstrom, welcher Methanol und Dimethylsulfid enthält, zugemischt, wodurch die Reaktionseingangstemperatur auf 450°C absinkt. Vor dem zweiten und den folgenden Reaktoren wird weiteres Methanol, teilweise als Flüssigkeit und teilweise als Gas in den Gasstrom gespritzt.

Die DE-C 11 34 368 betrifft die Verwendung eines Rohrbündelreaktors für die Herstellung von Methylmercaptan. Der Reaktor besteht aus einem zylindrischen Behälter, in dem die Katalysatorröhren parallel zueinander angeordnet sind. Die Katalysatorröhren sind unten und oben mit Rohrabdeckplatten, wie bei Rohrbündelwärmeaustauschern verschweißt, wobei die Zwischenräume zwischen den Röhren mit wärmeleitender Flüssigkeit gefüllt sind. Jede Katalysatorröhre ist an ihrem unteren Ende mit einem Sieb versehen, welches den teilchenförmigen Katalysator trägt. Die DE 196 54 515 betrifft ein Verfahren zur Herstellung von Methylmercaptan, bei dem die zur Verdampfung des Methanols benötigte Energie zum Teil durch Nutzung der Verdichtungswärme des Schwefelwasserstoffgases sowie durch den Wärmeinhalt des den Reaktor verlassenden Produktgases aufgebracht wird. Die Reaktionswärme wird hierbei genutzt, um das Eduktgasgemisch unter Zuhilfenahme eines externen Gaserhitzters auf die Reaktionstemperatur zu erwärmen.

Die Wirtschaftlichkeit des Gesamtprozesses hängt entscheidend von der Umsetzung des Eduktgasgemisches in einem geeigneten Druckreaktor und der Vorbereitung dieses Gasgemisches ab. Z.B. werden große elektrische Leistungen für den Betrieb der Verdichter und von Heiz- und Kühlkreisläufen benötigt. Ferner stellen aufwendige Katalysatorwechsel in Rohrbündelreaktoren aufgrund langer Stillstandzeiten einen nicht zu vernachlässigenden Zeit-und Kostenfaktor dar.

Aufgabe der Erfindung ist die Bereitstellung eines wirtschaftlichen Verfahrens zur Herstellung von Methylmercaptan.

Gegenstand der Erfindung ist ein Verfahren zur kontinuierlichen katalysierten Herstellung von Methylmercaptan durch Umsetzung von Methanol und Schwefelwasserstoff in der Gasphase bei einer Temperatur von 200 bis 600°C, insbesondere 250 bis 500°C und einem Druck von 1,5 bis 40 bar, wobei man
a) die Gesamtmenge des Katalysators auf mindestens zwei, bevorzugt mindestens drei voneinander getrennte Zonen gleich oder ungleich aufteilt,
b) die erste dieser Zonen mit einem gasförmigen Gemisch enthaltend Methanol und Schwefelwasserstoff (Eduktgas) beaufschlagt,
c) zwischen der ersten, der zweiten und gegebenenfalls den weiteren Zonen Methanol in flüssiger Form und/oder gasförmig einspeist, und
d) das gebildete Methylmercaptan abtrennt, wobei
   das Gesamtmolverhältnis der eingesetzte Mengen von Schwefelwasserstoff und Methanol sich 1:1 bis 10:1, bevorzugt 1:1 bis 5:1, besonders bevorzugt 1,1:1 bis 3:1 beläuft.

Das gasförmige Gemisch aus Schwefelwasserstoff und Methanol (Eduktgas) enthält diese beiden Verbindungen im Molverhältnis von 1,1:1 bis 20:1, bevorzugt 1,1:1 bis 10:1, insbesondere 3:1 bis 10:1 und gegebenenfalls Nebenprodukte aus der Reaktion und Inertgase, wenn beispielsweise nichtumgesetzte Komponenten zusammen mit diesem Verbindungen aus dem Produktgasstrom abgetrennt und recycliert werden.

Die Verwendung dieses Eduktgases garantiert schon in dem ersten Katalysatorbett eine gute Durchmischung der Reaktanden und eine durch den hohen Umsatz erzeugte Reaktionswärme, die zur Verdampfung des nach der ersten Zone eingespeisten Methanols eingesetzt wird. Wird, wie nach dem Stand der Technik üblich (GB 14 17 532), Methanol schon vor der ersten Zone eingedüst, muss zusätzlich Verdampfungsenergie zugeführt werden.

Ebenso kann das zwischen die die Katalysatoren enthaltenden Zonen eingespeiste Methanol schwefelhaltige Edukte oder Produkte enthalten, besteht aber im wesentlichen (im allgemeinen > 90 Mol.%) aus Methanol.

Bevorzugt wird ein Betriebsdruck von 2,5 bis 25 bar eingestellt.

Die Verdichtung der Eduktgase auf den Betriebsdruck wird ein- oder mehrstufig durchgeführt.

In der ersten Katalysator enthaltenden Zone liegt der Schwefelwasserstoff immer im Überschuss gegenüber Methanol vor.

Die Gesamtmengen des im Eduktgas und des zwischen den Zonen eingespeisten Methanols stehen im Verhältnis von 1:1 bis 1:10, bevorzugt 1:2 bis 1:7.

Bevorzugt setzt man Hordenreaktoren ein, die 2 bis 25 Katalysatorbetten (Zonen), insbesondere 3 bis 10, bevorzugt 3 bis 8 enthalten.

Dabei können auch mindestens zwei dieser Reaktoren miteinander verbunden werden.

Hordenreaktoren erlauben die direkte Zudosierung von gasförmigem und flüssigem Methanol, Schwefelwasserstoff oder ein unter anderem Methanol und Schwefelwasserstoff enthaltendes Eduktgasgemisch zwischen den Katalysatorbetten (Horden), wobei die in den Horden freiwerdende Reaktionswärme direkt für die Verdampfung des Methanols ausgenutzt wird, und die Temperatur des Gasgemisches vor Eintritt in die nächste Horde abfällt. Durch dieses Konzept kann auf leistungsstarke Methanolverdampfer verzichtet werden. Die Wirtschaftlickeit des Gesamtprozesses wird auch durch im Vergleich zu Rohrbündelreaktoren (mit Tausenden von zu befüllenden und entleerenden Einzelrohren), schnellere modulare Katalysatorwechsel in den Horden ermöglicht. So kann der Katalysator jeder Horde separat gewechselt werden. Dies ist insbesondere dann von Vorteil, wenn die Desaktivierung des Katalysators wie bei der Synthese von Methylmercaptan von den Konzentrationen der Reaktanden und somit vom Ort bzw. Reaktionsfortschritt abhängig ist. Weiterhin wird dadurch der Einsatz verschiedener Katalysatoren in einem Reaktor erleichtert.

Durch den Einsatz des Hordenreaktors ist speziell bei Reaktionen mit sehr starker Wärmetönung, wie beispielsweise der Synthese von Methylmercaptan aus Methanol und Schwefelwasserstoff über für den Prozess üblichen Katalysatoren, eine ausgezeichnete Temperaturkontrolle durch Wahl der Hordengröße und der Menge an eingespritzter Flüssigkeit möglich. Dadurch können bei der Synthese von Methylmercaptan hohe Übertemperaturen, die zu verringerten Ausbeuten und zu einer verstärkten Deaktivierung des Katalysators führen, vermieden werden.

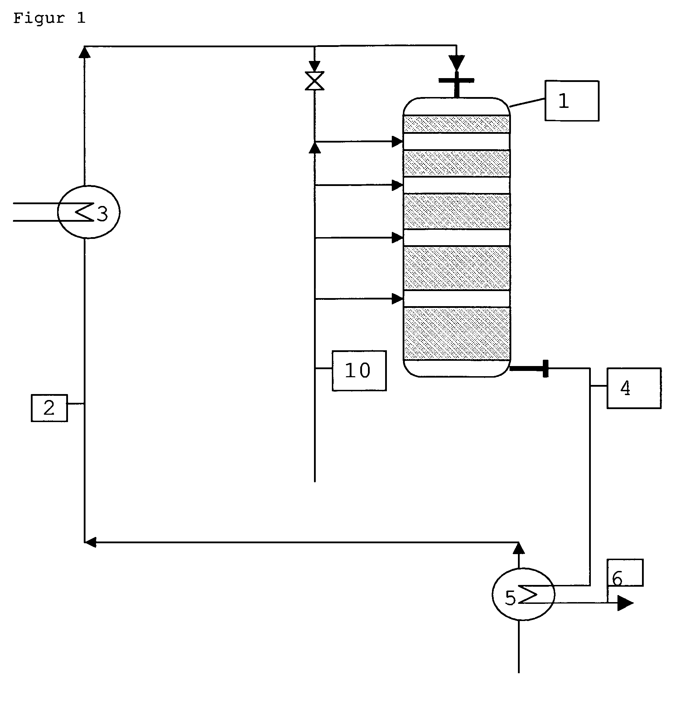

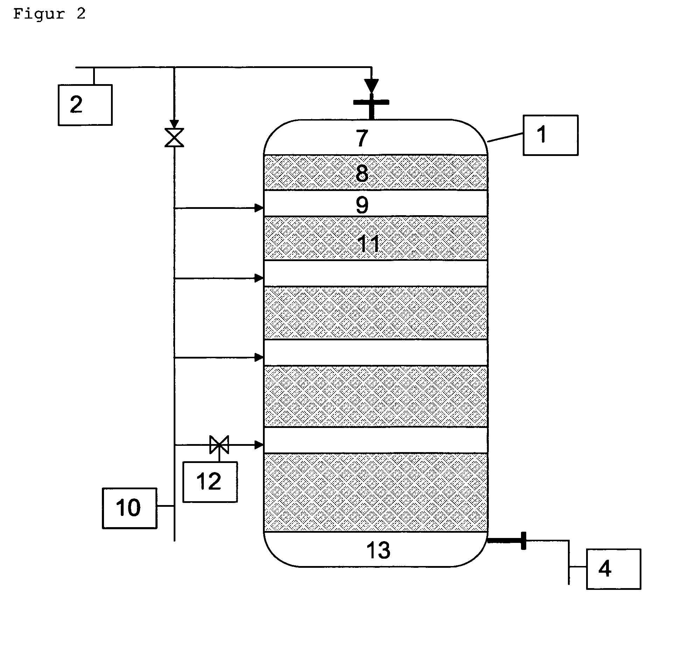

Die Figur 1 und Figur 2 dienen zur weiteren Erklärung des Verfahren unter Verwendung des bevorzugt eingesetzten Hordenreaktors.

Figur 1 zeigt ein Verfahrensschema des ersten Abschnittes des Herstellprozesses für Methylmercaptan.

Figur 2 zeigt eine Detaildarstellung eines in diesem Verfahren verwendeten Hordenreaktors.

Figur 1 zeigt ein Verfahrensschema für den ersten Abschnitt der Methylmercaptan-Herstellung, welcher die Eduktgasaufbereitung, die Umsetzung im Reaktor und die Kühlung des Produktgasgemisches umfasst. Die Umsetzung im Hordenreaktor 1 erfolgt an üblichen Katalysatoren, vorzugsweise auf Aluminiumoxid als Träger, welche bevorzugt mit Alkaliwolframat, insbesondere Cäsiumwolframat belegt sind. Die Katalysatoren werden in den Anmeldungen WO 2005/021491, DE 10 2004 77 39 und DE 10 2004 061 016 beschrieben.

Katalysatoren dieses Typs sind in der Lage, ein Eduktgasgemisch mit einem Molverhältnis von Schwefelwasserstoff zu Methanol von 1,5 : 1 bis 10,0 : 1 bei einem Betriebsdruck von 5-20 bar, einer Reaktionstemperatur von 280 - 450°C und bei einer Belastung mit einer Raumgeschwindigkeit GHSV von 300-2000 h⁻¹ mit einem Methanolumsatz und einer Selektivität von jeweils mehr als 90 % zu Methylmercaptan umzusetzen. Das erfindungsgemäße Verfahren erlaubt auch die Darstellung von Methylmercaptan unter Verwendung sonst üblicher Katalysatoren.

Aufgrund der sehr aktiven Katalysatoren zur Synthese von Methylmercaptan, die speziell halogenidfreie Alkaliwolframate, halogenidhaltige Alkaliwolframate oder bevorzugt halogenidfreie oder -haltige Cäsiumwolframate als Promotoren enthalten, ist eine ausgezeichnete Temperaturkontrolle zum Betrieb dieser Katalysatoren am Ausbeuteoptimum, ohne eine verstärkte Deaktivierung des Katalysators, befürchten zu müssen, erforderlich. Dies wird bei Verwendung dieser und anderer üblichen Katalysatoren und der erfindungsgemäßen Durchführung der Synthese im Hordenreaktor ermöglicht.

Das aus Methanol-Dampf, Schwefelwasserstoff und gegebenenfalls weiteren der oben genannten Komponenten bestehende Eduktgasgemisch 2 wird im Gaserhitzer 3 auf die Reaktoreingangstemperatur (Vortemperatur) von 100-350°C erwärmt. Mit dieser Temperatur gelangt das Eduktgasgemisch in den Hordenreaktor

Das Eduktgasgemisch ist auf Grund des Mengenanteils von Methanol und Schwefelwasserstoff nicht zu verwechseln mit einem Schwefelwasserstoffgas, das durch Recyclierung geringe Mengen an Methanol enthält.

Das Gasgemisch wird durch Verteileinrichtungen gleichmäßig über dem Katalysatorbett der ersten Reaktorhorde verteilt. Für eine bessere Wärmeübertragung ist das Katalysatorbett der ersten Reaktionshorde optional mit einer Schicht aus inerten, festen Füllkörpern zumindest im Bereich des Eintritts der Gasströmung überdeckt. Vorteilhafterweise werden hierfür beispielsweise Füllkörper Kugeln aus Keramik, Siliziumdioxid oder Aluminiumoxid verwendet. Der Hordenreaktor enthält im allgemeinen 2 bis 25 Katalysatorbetten, vorteilhafterweise werden 2 bis 10 Katalysatorbetten, bevorzugt 3-8 in einem Apparat untergebracht. Zwischen den Horden wird flüssiges oder gegebenenfalls gasförmiges Methanol, gegebenenfalls auch Schwefelwasserstoff oder das Eduktgasgemisch 2 in den Prozess eindosiert. Methanol wird vorzugsweise dem Prozess flüssig zwischen allen Horden oder einem Teil der Horden zugeführt. Dabei nutzt man die Reaktionswärme, die in der vor der Einspritzstelle befindlichen Horde frei wird, für die Verdampfung des Methanols und zur Kontrolle der Temperatur bei der stark exothermen Reaktion aus.

Als vorteilhaft hat sich eine Verlängerung der Bettlänge der Katalysatorhorden bzw. eine Erhöhung der Katalysatormenge von der ersten zur letzten Horde (Zone) in Strömungsrichtung erwiesen. Gegebenenfalls wird vor der letzten Horde kein Methanol eingespeist.

Zwischen den Katalysatorhorden befinden sich optional Vorrichtungen, wie zum Beispiel Statikmischer, geordnete oder ungeordnete Packungen, die einen turbulenten Strömungsverlauf und eine gleichmäßige Verteilung und Vermischung der Reaktanden ermöglichen. Bevorzugt wird das zwischen den Katalysatorhorden gegebenenfalls eindosierte Eduktgasgemisch und/oder das flüssige Methanol über einen Gasverteiler radial, tangential oder zonenweise über dem Katalysatorbett verteilt, so dass eine gleichmäßige turbulente Strömungsverteilung und vollständige Vermischung der Reaktanden resultiert. Die Vermischung der Reaktanden kann durch optionales Einbringen von Inertschichten aus Füllkörpern verbessert werden.

Die Katalysatorhorden sind vorteilhaft als Katalysatorbetten mit radialer, quadratischer oder polygonaler Geometrie gestaltet, wobei auch andere Geometrien möglich sind. Die Horden können einzeln mit Katalysator befüllt oder entleert werden. Vorzugsweise sind sie so gestaltet, dass sie dem Reaktor modular entnommen werden können. Alternativ kann jeweils eine lösbare oder unlösbare Verbindung bzw. Öffnung in der Reaktorwand zum einfachen Wechsel des Katalysators genutzt werden.

In einer weiteren Ausführungsform der Erfindung werden die Horden mit mindestens zwei unterschiedlichen Katalysatoren befüllt. Damit wird der Abhängigkeit der lokalen Ausbeute und lokalen Selektivität speziell bei der Synthese von Methylmercaptan in Abhängigkeit von den Konzentrationen der Reaktanden und somit dem Reaktionsfortschritt Rechnung getragen. Beispielsweise ist es bei der Synthese von Methylmercaptan vorteilhaft, die letzte Horde mit einem sehr aktiven Katalysator zu füllen, falls ein Vollumsatz an Methanol angestrebt ist. Soll die Umsetzung im Hinblick auf eine maximale Selektivität betrieben werden, so kann ein weniger aktiver, aber dafür sehr selektiver Katalysator in der letzten Horde eingesetzt werden. Der Hordenreaktor ermöglicht somit durch eine einfache, ortsabhängige Befüllung Flexibilität bei der Produktion von Methylmercaptan.

Das Produktgasgemisch 4 verlässt den Reaktor mit der Reaktionstemperatur der letzten Horde. Sein Wärmeinhalt kann im Wärmetauscher 5 für die Methanolverdampfung oder zur Erzeugung von Wasserdampf etc. ausgenutzt werden. Dabei kühlt sich das Produktgasgemisch auf etwa 150°C ab und wird als Mengenstrom 6 dem zweiten Verfahrensabschnitt zugeführt. Die Auftrennung des Produktgasgemisches in seine Komponenten wird im zweiten Verfahrensabschnitt der Methylmercaptan-Herstellung vorgenommen. Die Auftrennung kann nach verschiedenen, bekannten Verfahren erfolgen. Eine besonders vorteilhafte Auftrennung des Produktgasgemisches wird in der deutschen Patentschrift DE-C 196 54 516 beschrieben. Wichtig für die Wirtschaftlichkeit des Verfahrens ist die Rückführung des im zweiten Verfahrensabschnitt abgetrennten Schwefelwasserstoffes als Kreisgasstrom. Gleiches gilt für das vom Produktgasgemisch abgetrennte, bei der Umsetzung im Reaktor nicht vollständig verbrauchte Methanol, sowie für das im zweiten Verfahrensabschnitt gegebenenfalls verwendete Waschmethanol.

Fig. 2 zeigt die bevorzugte Ausführungsform des Reaktors, gemäß Anspruch 1. In dem Reaktor 1 sind n (n = 2-25) Katalysatorbetten untergebracht. Vorzugsweise werden 3-10 Katalysatorbetten (Horden) verwendet. Das Eduktgasgemisch 2 tritt über den Verteilerraum 7 in das erste Katalysatorbett 8 ein. Dieses erste Katalysatorbett ist optional in Strömungsrichtung des Eduktgases zunächst mit einer Schüttung aus inerten Materialien bedeckt. Beispielsweise werden Aluminiumoxidkugeln oder Keramik-Raschigringe als Inertmaterialien verwendet. In Anschluss an die Inertschicht befindet sich die Katalysatorschüttung. Durch die stark exotherme Bildung von Methylmercaptan steigt dabei die Temperatur in der adiabaten Horde stark an. Nach Verlassen der ersten Horde wird das Gasgemisch im Verteilerraum 9 mit flüssigem Methanol 10, Schwefelwasserstoff 10 oder optional dem Eduktgasgemisch 2 angereichert. Durch die Reaktionswärme der ersten Horde verdampft das flüssige Methanol ohne weitere Wärmezufuhr. Hierdurch sinkt die Temperatur der Gasmischung. Die Gasmischung strömt anschließend aus dem Verteilerraum 9 in das zweite Katalysatorbett 11, wobei Vorrichtungen im Verteilerraum 9 für eine turbulente Strömung und eine vollständige Vermischung der Reaktanden sorgen, die gleichmäßig auf die gesamte Fläche des zweiten Katalysatorbettes verteilt ist. Die Zufuhr von flüssigem Methanol oder optional Schwefelwasserstoff oder Eduktgasgemisch erfolgt analog an n-1, bevorzugt n-2 Einspritzstellen zwischen den folgenden Katalysatorbetten des Hordenreaktors. Optional kann auf eine Zufuhr von flüssigem Methanol, Schwefelwasserstoff oder Eduktgasgemisch vor dem letzten Katalysatorbett an der Einspritzstelle 12 verzichtet werden, um einen vollständigen Umsatz an Methanol in der Reaktion zu erhalten.

Nach Verlassen des Hordenreaktors wird das umgesetzte Gasgemisch über den Sammelraum 13 als Produktsgasstrom 4 der weiteren Verarbeitung zugeführt.

Somit kann in nur einem Reaktionsapparat inklusive eines integrierten direkten Wärmeaustausch ohne zusätzliche Wärmeträgermedien wie Salzschmelzen oder Dampf die Temperatur einer stark exothermen Reaktion ausgezeichnet kontrolliert werden.

Das in Fig. 1 dargestellte Verfahrensschema enthält die für die Durchführung des erfindungsgemäßen Verfahrens notwendigen Komponenten.

## Patentansprüche

1. Verfahren zur kontinuierlichen katalysierten Herstellung von Methylmercaptan durch Umsetzung von Methanol und Schwefelwasserstoff in der Gasphase bei einer Temperatur von 200 bis 600°C und einem Druck von 1,5 bis 40 bar, wobei man
a) die Gesamtmenge des Katalysators auf mindestens zwei voneinander getrennte Zonen aufteilt,
b) die erste dieser Zonen mit einem gasförmigen Gemisch, enthaltend Methanol und Schwefelwasserstoff beaufschlagt,
c) zwischen der ersten, der zweiten und gegebenenfalls den weiteren Zonen Methanol in flüssiger Form und/oder gasförmig einspeist, und
d) das gebildete Methylmercaptan abtrennt, wobei
e) das Gesamtmolverhältnis der eingesetzte Mengen von Schwefelwasserstoff und Methanol sich auf 1,1:1 bis 10:1, bevorzugt 1,1:1 bis 5:1 beläuft.

2. Verfahren gemäß Anspruch 1, **dadurch gekennzeichnet, dass** man einen Hordenreaktor mit 2 bis 25 Katalysatorbetten, bevorzugt 3 bis 10 Katalysatorbetten, einsetzt.

3. Verfahren gemäß den Ansprüchen 1 oder 2, **dadurch gekennzeichnet, dass** man die Katalysatorschüttungen in den Zonen mit Schüttungen aus inerten Materialien überschichtet.

4. Verfahren gemäß Anspruch 3, **dadurch gekennzeichnet, dass** man die in Strömungsrichtung erste Katalysatorzone und gegebenenfalls die letzte Zone ganz oder teilweise mit einem inerten Material beschichtet.

5. Verfahren gemäß den Ansprüchen 1 bis 4, **dadurch gekennzeichnet, dass** man in den Zonen mindestens zwei verschiedene Katalysatoren einsetzt.

6. Verfahren gemäß den Ansprüchen 1 bis 5, **dadurch gekennzeichnet, dass** die Katalysatormenge zwischen den Zonen variiert, insbesondere in Strömungsrichtung zunimmt.

7. Verfahren gemäß den Ansprüchen 1 bis 6, **dadurch gekennzeichnet, dass** man die Gesamtmenge des eingesetzten Schwefelwasserstoffs als gasförmiges Gemisch von Methanol und Schwefelwasserstoff einsetzt.

8. Verfahren gemäß den Ansprüchen 1 bis 6, **dadurch gekennzeichnet, dass** man in den Zwischenraum zwischen einer oder mehreren der ersten und zweiten oder weiteren Zonen Schwefelwasserstoff oder Eduktgas einspeist.

9. Verfahren gemäß den Ansprüchen 1 oder 2, **dadurch gekennzeichnet, dass** das molare Verhältnis von Schwefelwasserstoff zu Methanol in dem Eduktgas 1,1:1 bis 20:1, bevorzugt 1,1:1 bis 10:1, insbesondere 3:1 bis 10:1 beträgt.

10. Verfahren gemäß den Ansprüchen 1 oder 2, **dadurch gekennzeichnet, dass** man das Methanol in die Zwischenräume zwischen den Zonen in gleichen Mengen einspeist.

11. Verfahren gemäß den Ansprüchen 1 oder 2, **dadurch gekennzeichnet, dass** man das Methanol in die Zwischenräume zwischen den Zonen, in ungleichen Mengen einspeist.

12. Verfahren gemäß den Ansprüchen 1 oder 2, **dadurch gekennzeichnet, dass** man die letzte Katalysator enthaltende Zone nicht mit Methanol beaufschlagt.

13. Verfahren gemäß den Ansprüchen 2 bis 12, **dadurch gekennzeichnet, dass** die Katalysatorbetten in wechselnden Richtungen mit Methanol und Schwefelwasserstoff durchströmt werden.

14. Verfahren gemäß den Ansprüchen 1 bis 13, **dadurch gekennzeichnet, dass** man als Katalysatoren Alkaliwolframate oder halogenidhaltige Alkaliwolframate einsetzt.

15. Verfahren gemäß Anspruch 14, **dadurch gekennzeichnet, dass** man halogenidfreie oder halogenidhaltige Cäsiumwolframate einsetzt.

16. Verfahren gemäß den Ansprüchen 1 bis 15, **dadurch gekennzeichnet, dass** man das Reaktionsgemisch zwischen den Zonen jeweils vollständig vermischt, durch Einbringen von inerten Füllkörpern und/oder Vorrichtungen vermischt, die einen turbulenten Strömungsverlauf und eine gleichmäßige Verteilung und Vermischung der Reaktanden ermöglichen.

17. Verfahren gemäß den Ansprüchen 1 bis 16, **dadurch gekennzeichnet, dass** die Katalysatorhorden als Katalysatorbetten mit radialer, quadratischer oder polygonaler Geometrie gestaltet sind und die Horden einzeln mit Katalysator befüllt oder entleert werden können, wobei sie optional so gestaltet sind, dass sie dem Reaktor modular entnommen werden können.

18. Verfahren gemäß den Ansprüchen 1 bis 17, **dadurch gekennzeichnet, dass** jeweils mindestens eine lösbare oder unlösbare Verbindung oder Öffnung in der Reaktorwand zum einfachen Wechsel des Katalysators in den einzelnen Horden genutzt wird.

## Claims

1. Process for the continuous catalysed production of methyl mercaptan by reaction of methanol and hydrogen sulphide in the gas phase at a temperature of 200 to 600°C and a pressure of 1.5 to 40 bar, where
a) the total amount of the catalyst is distributed to at least two zones which are separate from one another,
b) the first of these zones is supplied with a gaseous mixture comprising methanol and hydrogen sulphide,
c) between the first, the second and optionally the further zones methanol is fed in in liquid and/or gaseous form, and
d) the methyl mercaptan formed is separated off,
e) the total molar ratio of the amounts of hydrogen sulphide and methanol employed amounting to 1.1:1 to 10:1, preferably 1.1:1 to 5:1.

2. Process according to Claim 1, **characterized in that** a grid reactor having 2 to 25 catalyst beds, preferably 3 to 10 catalyst beds, is employed.

3. Process according to Claim 1 or 2, **characterized in that** the catalyst packings in the zones are covered with a layer of the packings of inert materials.

4. Process according to Claim 3, **characterized in that** the first catalyst zone in the flow direction and optionally the last zone are completely or partly coated with an inert material.

5. Process according to Claims 1 to 4, **characterized in that** at least two different catalysts are employed in the zones.

6. Process according to Claims 1 to 5, **characterized in that** the amount of catalyst between the zones varies, in particular increases in the flow direction.

7. Process according to Claims 1 to 6, **characterized in that** the total amount of hydrogen sulphide employed is employed as a gaseous mixture of methanol and hydrogen sulphide.

8. Process according to Claims 1 to 6, **characterized in that** hydrogen sulphide or starting gas is fed into the intermediate space between one or more of the first and second or further zones.

9. Process according to Claim 1 or 2, **characterized in that** the molar ratio of hydrogen sulphide to methanol in the starting gas is 1.1:1 to 20:1, preferably 1.1:1 to 10:1, in particular 3:1 to 10:1.

10. Process according to Claim 1 or 2, **characterized in that** the methanol is fed into the intermediate spaces between the zones in equal amounts.

11. Process according to Claim 1 or 2, **characterized in that** the methanol is fed into the intermediate spaces between the zones in unequal amounts.

12. Process according to Claim 1 or 2, **characterized in that** the last catalyst-containing zone is not supplied with methanol.

13. Process according to Claims 2 to 12, **characterized in that** the catalyst beds are flowed through in variable directions with methanol and hydrogen sulphide.

14. Process according to Claims 1 to 13, **characterized in that** the catalysts employed are alkali metal tungstates or halide-containing alkali metal tungstates.

15. Process according to Claim 14, **characterized in that** halide-free or halide-containing caesium tungstates are employed.

16. Process according to Claims 1 to 15, **characterized in that** the reaction mixture is in each case completely mixed between the zones by introduction of inert packing materials and/or devices which make possible a turbulent flow course and a uniform distribution and mixing of the reactants.

17. Process according to Claims 1 to 16, **characterized in that** the catalyst grids are designed as catalyst beds having radial, square or polygonal geometry and the grids can be individually filled with catalyst or emptied, their design optionally being such that they can be removed from the reactor in modular form.

18. Process according to Claims 1 to 17, **characterized in that** in each case at least one detachable or undetachable connection or opening in the reactor wall is utilized for the simple exchange of the catalyst in the individual grids.

## Revendications

1. Procédé pour la préparation continue, catalysée de méthylmercaptan par transformation de méthanol et de sulfure d'hydrogène en phase gazeuse à une température de 200 à 600°C et à une pression de 1,5 à 40 bars, dans lequel
a) on répartit la quantité totale du catalyseur sur au moins deux zones séparées l'une de l'autre,
b) on alimente la première des ces zones par un mélange gazeux, contenant du méthanol et du sulfure d'hydrogène,
c) on injecte entre la première, la deuxième et le cas échéant les autres zones, du méthanol sous forme liquide et/ou gazeuse, et
d) on sépare le méthylmercaptan formé,
e) le rapport molaire total des quantités utilisées de sulfure d'hydrogène et de méthanol s'élevant à 1,1:1 jusqu'à 10:1, de préférence à 1,1:1 jusqu'à 5:1.

2. Procédé selon la revendication 1, **caractérisé en ce qu'**on utilise un réacteur à plateaux comprenant 2 à 25 lits catalytiques, de préférence 3 à 10 lits catalytiques.

3. Procédé selon les revendications 1 ou 2, **caractérisé en ce qu'**on recouvre les catalyseurs en vrac dans les zones par des matériaux inertes en vrac.

4. Procédé selon la revendication 3, **caractérisé en ce qu'**on recouvre la première zone catalytique dans le sens de l'écoulement et le cas échéant la dernière zone, totalement ou partiellement, par un matériau inerte.

5. Procédé selon les revendications 1 à 4, **caractérisé en ce qu'**on utilise dans les zones au moins deux catalyseurs différents.

6. Procédé selon les revendications 1 à 5, **caractérisé en ce que** la quantité de catalyseur varie entre les zones, en particulier augmente dans le sens de l'écoulement.

7. Procédé selon les revendications 1 à 6, **caractérisé en ce qu'**on utilise la quantité totale du sulfure d'hydrogène utilisé sous forme de mélange gazeux de méthanol et de sulfure d'hydrogène.

8. Procédé selon les revendications 1 à 6, **caractérisé en ce qu'**on injecte du sulfure d'hydrogène ou du gaz de départ dans l'espace intermédiaire entre une ou plusieurs zones de la première et de la deuxième ou des autres zones.

9. Procédé selon les revendications 1 ou 2, **caractérisé en ce que** le rapport molaire de sulfure d'hydrogène à méthanol dans le gaz de départ vaut 1,1:1 à 20: l, de préférence 1,1:1 à 10:1, en particulier 3:1 à 10:1.

10. Procédé selon les revendications 1 ou 2, **caractérisé en ce qu'**on injecte le méthanol dans les espaces intermédiaires entre les zones en des quantités identiques.

11. Procédé selon les revendications 1 ou 2, **caractérisé en ce qu'**on injecte le méthanol dans les espaces intermédiaires entre les zones, en des quantités différentes.

12. Procédé selon les revendications 1 ou 2, **caractérisé en ce qu'**on n'alimente pas de méthanol dans la dernière zone contenant un catalyseur.

13. Procédé selon les revendications 2 à 12, **caractérisé en ce que** les lits catalytiques sont traversés par des flux de méthanol et de sulfure d'hydrogène dans des sens alternants.

14. Procédé selon les revendications 1 à 13, **caractérisé en ce qu'**on utilise, comme catalyseurs, des tungsténates de métal alcalin ou des tungsténates de métal alcalin contenant des halogénures.

15. Procédé selon la revendication 14, **caractérisé en ce qu'**on utilise des tungsténates de césium exempts d'halogénures ou contenant des halogénures.

16. Procédé selon les revendications 1 à 15, **caractérisé en ce qu'**on mélange à chaque fois complètement le mélange réactionnel entre les zones, par introduction de corps de remplissage inertes et/ou de dispositifs, qui permettent un écoulement turbulent et une répartition et un mélange réguliers des réactifs.

17. Procédé selon les revendications 1 à 16, **caractérisé en ce que** les plateaux catalytiques sont réalisés sous forme de lits catalytiques présentant une géométrie radiale, carrée ou polygonale et les plateaux peuvent être remplis de catalyseur ou vidés individuellement, en étant éventuellement réalisés de manière telle qu'ils peuvent être enlevés sous forme de module du réacteur.

18. Procédé selon les revendications 1 à 17, **caractérisé en ce qu'**on exploite à chaque fois au moins un assemblage amovible ou non ou une ouverture dans la paroi du réacteur pour un remplacement simple du catalyseur dans les différents plateaux.
